# EUROPEAN PATENT SPECIFICATION

(11) **EP 3 710 065 B1**
(45) Date of publication and mention of the grant of the patent: **21.08.2024**
(21) Application number: 18807126.0
(22) Date of filing: 30.10.2018
(51) Int. Cl.: A61K 47/68, A61K 47/69

(54) **BIOMIMETIC NON-IMMUNOGENIC NANOASSEMBLY FOR THE ANTITUMOR THERAPY**
BIOMIMETISCHE NICHTIMMUNOGENE NANOANORDNUNG FÜR DIE ANTITUMORTHERAPIE
NANOENSEMBLE BIOMIMÉTIQUE NON IMMUNOGÈNE POUR LA THÉRAPIE ANTITUMORALE

(30) Priority: 13.11.2017 IT 201700129243
(43) Date of publication of application: 23.09.2020
(73) Proprietor: Politecnico Di Torino, 10129 Torino (IT)
(72) Inventor: CAUDA, Valentina Alice, 10129 Torino (IT); CANAVESE, Giancarlo, 10129 Torino (IT); LIMONGI, Tania, 10129 Torino (IT); GARINO, Nadia, 10129 Torino (IT); LAURENTI, Marco, 10129 Torino (IT); RACCA, Luisa, 10129 Torino (IT); ANCONA, Andrea, 10129 Torino (IT); CANTA, Marta, 10129 Torino (IT); DUMONTEL, Bianca, 10129 Torino (IT)
(74) Representative: Praxi Intellectual Property Milano
(86) International application number: PCT/IB2018/058476
(87) International publication number: WO 2019/092550

(56) References cited:
- WO-A2-2009/039508
- WO-A2-2015/110957
- US-A1- 2017 232 115

## Description

The present invention relates to a nanoassembly for inducing apoptosis in cancer cells comprising: (i) a core comprising at least one nanoparticle of a nanostructured and semiconductor metal oxide, said nanoparticle being monocrystalline or polycrystalline; (ii) a shell formed by a double phospholipid layer, proteins and other molecules, said shell consisting of an extracellular biovesicle derived from cells of the same organism which said cancer cells belong to, said core being enclosed inside said shell; and (iii) a plurality of targeting molecules of said cancer cells, said molecules being anchored to the external surface of said at least one biovesicle.

The present invention also relates to a medicament comprising such nanoassembly for use in antitumor therapy, a method for the production of said nanoassembly, and a kit for use of the nanoassembly as a contrast medium comprising an injectable solution of said nanoassembly, also disclosed but not part of the invention as claimed are means for the emission of electromagnetic radiation with which radiating said nanoassembly; means for detecting the fluorescence radiation emitted by said nanoassembly because of the irradiation performed by said emitting means.

Nowadays, cancer results being the second major cause of death in the world and surpassed only by cardiocirculatory diseases. The International Agency for Research on Cancer (IARC) has estimated that in 2012 occurred 14.1 million new cases of cancer that have been associated with 8.2 million deaths worldwide. In 2030, because of the ageing of the population of the world, a further increase of newly diagnosed cases is expected, equal to approximately 21.7 million with a mortality rate of 13 million people.

The cancer therapies known today are based on chemotherapy, radiotherapy, the surgical removal of the tumor mass or on a combination of these. Such therapies are, unfortunately, characterised by a reduced selectivity on the tumor tissue with respect to healthy cells and tissues and, therefore, give rise to major side effects, both at the systemic and specific level, on various organs. In addition, very often cancers develop a resistance to chemotherapeutic drugs administered during the first treatment cycles, thus making the pathology no longer curable. The more recent introduction of immunotherapy, in association with chemotherapy, has led to improvements in the fight against cancer, however, the monoclonal antibodies, so far identified as an effective and selective therapy, are valid only for certain types of cancer.

To overcome these issues, in recent decades in the context of the so-called nanomedicine, various inventions have been proposed, consisting of nanomaterials suitably engineered so that to perform a controlled and programmable release of chemotherapeutic drugs, with a certain selectivity toward cancer cells. WO2009039508A2, for example, discloses ZnO nanoparticles enhanced with targeting molecules (i.e. monoclonal antibodies, peptides and small molecules) for inducing apoptosis in the cancer cells. US2017232115A1 describes nanoassembly (i.e. protocell) for treating cancer, comprising: a core of semiconductor quantum dots, or metallic nanoparticles, a shell formed by a double lipid layer and proteins, targeting molecules ad at least one agent which facilitates cancer cell death (such as a traditional small molecule, a macromolecular cargo (e.g. siRNA, shRNA other micro RNA, or a protein toxin such as ricin toxin A-chain or diphtheria toxin A-chain) and/or DNA. WO2015110957A2 discloses a nanoassembly (i.e. hybridosome) comprising: at least one biocompatible delivery module (BDM), such as biovesicle; at least one engineered drug encapsulation module (EDEM) provided with at least one tunable fusogenic moiety. The EDEM and the BDM are united so that a common internal space is formed. Such internal space contains contains at least one bioactive agents originating from a BDM secreted in vivo (e.g. endogenous polynucleotides, enzymes or polypeptides) and at least one bioactive agent encapsulated in an EDEM manufactured in vitro. Such nanomaterials, despite being able to improve the performance of traditional chemotherapies, are still strongly affected by non-negligible limitations. Because of these, the introduction in clinical practice, and therefore on the market, is still severely restricted. In particular, despite being possible with the known nanomaterials to obtain at least a certain selectivity of treatment toward cancer cells, the release of the chemotherapeutic drug in other areas of the body, that are different from those that need to be treated, still remains non-negligible.

The object of the present invention is, therefore, to further reduce the side effects on healthy cells and tissues, with respect to what is already possible using the nanomaterials currently used for the controlled release of a chemotherapeutic drug. This object is achieved by the present invention both substantially increasing the specificity and selectivity toward target cells, and by means of the complete elimination of the chemotherapeutic drugs. These latter, in particular, are replaced, in the present invention, by nanocrystals of semiconductor metal oxides (e.g. zinc oxide) which, thanks to their properties of semiconductivity and to the crystalline defects caused by their nanostructuring, are capable of generating radical species and metal ions that, if released inside the cells, have toxic properties.

The improvement of specificity and selectivity is obtained, on the other hand, by means of coating the semiconductor nanocrystals with extracellular biovesicoles (e.g. exosomes), on whose external surface molecules, capable of delivering the nanoassembly itself to cancer cells, are connected. In particular, the nanoassembly of the present invention comprises:
- a core comprising at least one nanoparticle of a nanostructured and semiconductor metal oxide, said nanoparticle being monocrystalline or polycrystalline;
- a shell formed by a double phospholipid layer, proteins and other molecules, said shell consisting of an extracellular biovesicle derived from cells of the same organism which said cancer cells belong to, said core being enclosed inside said shell; and
- a plurality of targeting molecules of cancer cells, in which the apoptosis is desired to be induced, said molecules being anchored to the external surface of said shell.

For the purposes of the present description, the expression "targeting molecules" of cancer cells means molecules for targeting cancer cells and coupling the nanoassembly with cancer cells in which apoptosis is desired to be induced. These molecules can be, for example, monoclonal antibodies, peptides, folic acid, growth factors and sugars. With the proviso that any semiconductor and nanostructured metal oxide, thanks to its ability to generate radical species toxic to cancer cells, can be used to achieve the purposes of the present invention, the nanoassembly of the present invention preferably comprises a core of zinc oxide nanostructured in crystals of spherical shape and in wurtzite phase. The shell enclosing the core of the nanocrystal consists of an extracellular biovesicole which can be chosen from the group comprising an exosome, an ectosome, a connectosome, an oncosome and an apoptotic body. The diameter of said at least one nanocrystal is furthermore preferably comprised between 5 nm and 100 nm, and the diameter of said biovesicle is preferably comprised between 30 nm and 300 nm. Here, it is specified that the cytotoxicity of the zinc oxide is known in the current state of the art (WO2011022350A1, US8187638B2, US20120184495A1, US20150335744A1, WO2007109290A2), but its use as a core enclosed by a shell derived from an extracellular biovesicole is completely unknown.

The mechanism with which said nanoassembly performs its antitumor function is the following: thanks to the molecules present on the coating of the nanoassembly, the nanoassembly interacts ("cell targeting") with the phospholipidic layer of the membrane of the cancer cells by delivering in a selective manner the nanocrystals of zinc oxide therein. The nanocrystals once present in cancer tissue and entered into the cancer cells, due to their more acid environment, release highly cytotoxic ionic species Zn²⁺ and radical oxygen species.

Another problem which affects the nanomaterials known so far for cancer therapy consists in their immunogenicity, i.e. in their ability to induce an immune reaction of the organism that, recognizing these nanomaterials as "outsiders", reduces their therapeutic efficacy, or even neutralizes their effect. The activation of the immune system (both innate and acquired) can also bring in some cases even to more serious consequences at the systemic level. Typically, the immunogenic nanomaterials, if they are also not hemocompatible, can induce the formation of clots and thrombi in blood, haemolysis, and in the most severe cases, heart attacks or strokes.

A second object of the present invention is, therefore, to provide a nanoassembly for antitumor therapy that is non-immunogenic, i.e. that does not generate an immune reaction by the organism.

This object is achieved by the present invention thanks to the particular coating of a double phospholipid layer used to enclose the nanocrystals of a semiconductor oxide.

In particular, the presence of a double phospholipid layer, derived from the same organism in which will be reused for the antitumor therapy, reduces the likelihood of adverse immune reactions.

In addition to the aforesaid issues, nanomaterials known today have the further disadvantage of being often toxic, and their accumulation in the typical target organs (kidneys, liver, spleen, etc.) can lead to problems of poisoning of the latter.

A third object of the present invention is, therefore, to provide a nanoassembly for use in the antitumor therapy, that is not toxic in target organs where usually drugs accumulate, such as kidneys, liver and spleen.

This object is achieved by the present invention in that the zinc oxide, which constitutes the core of the nanoassembly, is enclosed in a lipid shell, and exits therefrom only after "targeting" the cancer cells. The nanoassembly is, therefore, per se, non-toxic, becoming so only after the coupling with the cancer cells.

A further object of the present invention is, at last, to provide a nanoassembly for antitumor therapy that could also be used as a means for diagnostic imaging, so as to view the changes in the disease during the progress of the therapeutic protocol.

This object is achieved by the present invention thanks to the fact that the zinc oxide, which constitutes the core of the nanoassembly, if suitably excited by light sources with wavelengths in the ultraviolet spectrum, is able to re-emit light astride of the ultraviolet and the green region of the visible spectrum. The nanoassembly object of the present invention can, therefore, be used as a theranostic tool capable of performing both therapeutic and diagnostic functions at the same time. As will be explained in more detail below, the zinc oxide nanocrystals, if excited by a radiation in the ultraviolet spectrum (100 - 400 nm), emit a fluorescence radiation with a wavelength ranging between 380 nm and 550 nm, partially included in the ultraviolet spectrum (100 - 400 nm), and partially included in the visible light spectrum, and in particular in the green light one (495 - 570 nm). The present invention has, therefore, as an object a kit comprising:
- an injectable solution comprising said nanoassembly;
- emitting means electromagnetic radiations in the ultraviolet spectrum with which radiating said nanoassembly;

- means for detecting the fluorescence radiation emitted by said nanoassembly because of the irradiation performed by said emitting means.

To improve the detectability of the fluorescence radiation emitted by the zinc oxide, the zinc oxide can be doped with an element amplifying such radiation. It is also possible to dope the zinc oxide with an element which modifies the spectrum of frequencies of the fluorescence radiation of said zinc oxide, for example, up to include the frequencies of the infrared spectrum. In the case in which the metal oxide used as the core of nanoassembly is different from the zinc oxide and does not possess fluorescence properties, obviously it is possible to combine such oxide with a dye element (with fluorescence properties), which, if suitably excited by electromagnetic radiation, is capable of re-emitting them at wavelengths higher than that of the exciting radiations. A further alternative, valid both in the case where a zinc oxide core is used and in case a core consisting of another nanostructured semiconductor oxide is used, consists in doping such oxide with a paramagnetic or diamagnetic material, said element being able to be detected by means of nuclear magnetic resonance.

These and further objects of the present invention will be made clearer on reading the following detailed description of a preferred embodiment of the present invention, by way of example and not limitation of the more general claimed concepts, as well as by way of examples concerning experimental tests performed on the present invention.

The following description refers to the accompanying figures, wherein:
- Figure 1 is an exemplary diagram of the nanoassembly object of the present invention;
- Figure 2a is a scanning electron microscopy image of zinc oxide nanocrystals synthesized according to the method of the present invention;
- Figure 2b is a transmission electron microscopy image of zinc oxide nanocrystals synthesized according to the method of the present invention;
- Figure 2c is an image of the electron diffraction pattern in transmission electronic microscopy of zinc oxide nanocrystals synthesized according to the method of the present invention;
- Figure 2d shows a diffractogram obtained by X-ray diffraction (with radiation source Cu K alpha-= 0.15418 nm) on the zinc oxide nanocrystals synthesized according to the method of the present invention;
- Figure 3a is a field emission scanning electron microscopy (FESEM) image of zinc oxide nanocrystals synthesized according to the method of the present invention;
- Figure 3b is a graph of the distribution of the diameters of the zinc oxide nanocrystals synthesized according to the method of the present invention, said nanocrystals being dispersed in an ethanol solution;

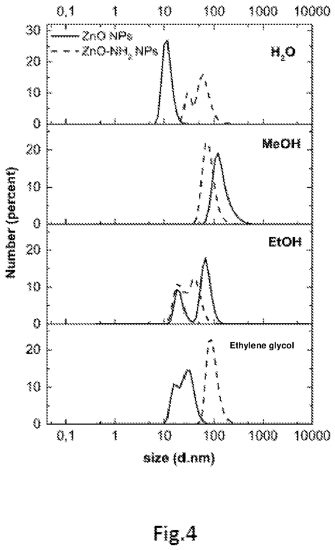

- Figure 4 summarizes the distribution graphs of the size of the nanocrystals synthesized according to the method of the present invention, said graphs being obtained by means of the "Dynamic Light Scattering" (DLS) technique. In particular, solid line curves refer to the ZnO nanocrystals, the dashed line curves relate to ZnO functionalized with amino-propyl groups (ZnO-NH₂), the first line refers to nanocrystals dispersed in water (H₂O), the second line to nanocrystals dispersed in methanol (MeOH), the third line to nanocrystals dispersed in ethanol (EtOH), and finally the fourth line refers to nanocrystals dispersed in ethylene glycol (EG);
- Figure 5a is a FESEM image of exosomes extracted according to the method of the present invention from epithelial oral cancer cells (KB type) in adhesion;
- Figure 5b is a graph, obtained by means of "Nanoparticle Tracking Analysis" (NTA) of the distribution of diameters of exosomes extracted according to the method of the present invention from cancer cells derived from an epithelial oral cancer (KB type) in adhesion;
- Figure 6a is a FESEM image of exosomes extracted according to the method of the present invention from type B lymphomatous cancer cells (Daudi type) in suspension;
- Figure 6b is a graph obtained by means of NTA analysis of the distribution of diameters of exosomes extracted with the method of the present invention from type B lymphomatous cancer cells (Daudi type);
- Figure 7 summarizes the distribution graphs of the size of the exosomes extracted by type KB cells, of the size of zinc oxide nanocrystals and complete nanoassemblies obtained according to the method of the present invention, said graphs being obtained by means of the "Dynamic Light Scattering" (DLS) technique. In particular, the solid line curve refers to the nanoassemblies, the dashed line curve with alternating points and dashes refers to exosomes, and the dashed line curve refers to zinc oxide crystals;
- Figure 8a shows the measurements of the concentrations of zinc, calcium and phosphate ions released by zinc oxide (ZnO), zinc oxide functionalized with amino-propyl groups (ZnO-NH₂), and zinc oxide coated with a phospholipid layer, in simulated inorganic plasma (SBF, Simulated Body Fluid). The graphs are obtained by means of "Inductive Coupled Plasma" (ICP) techniques;
- Figure 8b shows the measurements of the concentrations of zinc, calcium and phosphate ions released by zinc oxide (ZnO), zinc oxide functionalized with amino-propyl groups (ZnO-NH₂) and zinc oxide coated with a phospholipid layer, in a cell culture medium (EMEM). The graphs were obtained by inductively coupled plasma mass spectrometry (ICP-MS) (ICP);
- Figure 9 shows the fluorescence emission in the wavelength at approximately 400 nm and 550 nm, obtained by exciting the zinc oxide nanocrystals with a monochromatic source at 230 nm and 300 nm, respectively;
- Figure 10a shows the result of cell viability tests after 5 hours (H) of line KB cancer cell exposure to different concentrations of zinc oxide in a cell culture medium. The graph shows, in particular, the percentage of live cells with respect to the control according to the concentration of zinc oxide; and
- Figure 10b shows the result of cell viability tests after different exposure times (24, 48 and 72 hours (H)) of line KB cancer cells to different concentrations of zinc oxide in a cell culture medium. The graph shows, in particular, the percentage of live cells in comparison with respect the control as a function of the concentration of zinc oxide.

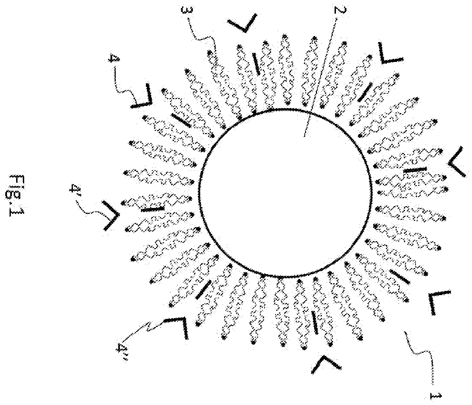

With reference to Figure 1, in a preferred embodiment, the present invention regards a biomimetic nanoassembly (1) comprising:
- a core (2) comprising at least one zinc oxide nanocrystal, said nanocrystal being spherical-shaped and in wurtzite phase;
- a shell (3) formed by a double phospholipid layer and other molecules, including proteins, derived from an exosome, said core (2) being enclosed inside said shell (3); and
- a plurality of monoclonal antibodies (4, 4', 4"), said antibodies (4, 4', 4") being anchored to the external membrane of said exosome.

The diameter of said at least one nanocrystal is comprised between 5 nm and 100 nm, and the diameter of said exosome is comprised between 30 nm and 300 nm.

The nanoassembly (1) of the present invention may optionally comprise also a compound having an anti-inflammatory action and/or a compound having a chemotherapeutic action as an adjuvant of the cytotoxic action of zinc oxide. In addition, the zinc oxide, *per se* capable of emitting a fluorescence radiation in a band astride the spectrum of ultraviolet and visible green light spectra when excited by ultraviolet radiations, can be doped with an element that amplifies this fluorescence radiation or with an element that changes the frequency spectrum of this fluorescence radiation. Actually, with a suitable doping, it is possible to broaden the spectrum to include the frequency of the infrared spectrum.

In such context, it is specified here that a kit for the use of the nanoassembly (1) as a contrast medium forms an object of the present invention, said kit comprising:
- an injectable solution comprising said nanoassembly (1);
- means for emitting electromagnetic radiations in the ultraviolet spectrum by which radiating said nanoassembly (1);
- means for detecting the fluorescence radiation emitted by said nanoassembly (1) following the irradiation performed by said emitting means.

The means for the detection of fluorescence radiation comprise a camera or an optoelectronic device or a spectrophotometer configured to detect radiations in the visible light spectrum and/or a camera or an optoelectronic device or a spectrophotometer configured to detect radiations in the infrared spectrum.

Also, a method for the production of the nanoassembly (1) described above is an object of the present patent application, said method comprising the steps of:
- synthesis of the zinc oxide;
- *in vitro* culture of a plurality of cancer cells for a period ranging between 24 and 48 hours;
- extraction of a plurality of biovesicles from said plurality of cancer cells, said biovesicles having a diameter comprised between 30 nm and 300 nm;
- coupling of the biovesicles with the zinc oxide; and
- separation of the biovesicles coupled to the zinc oxide, remained non-coupled biovesicles and remained non-coupled zinc oxide.

The synthesis of zinc oxide can be by wet-chemical synthesis, such as sol-gel or hydrothermal or solvothermal synthesis, or provide for the use of a microwave source, or an ultrasound source, or a solid-state synthesis of grinding or crushing with a mill. The coupling between biovesicles and zinc oxide provides for the incubation of both in a solution obtained by mixing water and a phosphate-buffered saline (PBS) with a volume ratio of one to one, for a period comprised between 30 minutes and two hours and thirty minutes, at a temperature comprised between the room temperature and 37 °C, in static conditions or under mechanical stirring generated by an orbital stirrer with a number of rotations comprised between 50 and 350 rpm. At the separation between biovesicoles coupled to zinc oxide, the remained non-coupled biovesicoles and the remained non-coupled zinc oxide is carried out by means of centrifugation with an acceleration greater than 10000 g for at least five minutes, followed by a washing in a solution of water and phosphate-buffered saline with a volume ratio of one to one. Finally, also a medicament or molecule comprising the nanoassembly (1), described above for use in antitumoral therapy and for use in the prevention of the immune response from the human organism treated with an antitumor therapy, is an object of the present invention. It is, therefore, possible to design and implement a treatment protocol, or a treatment method of cancers based on the administration of a drug comprising the nanoassembly (1) object of the present invention.

### EXAMPLE 1

### Synthesis of zinc oxide.

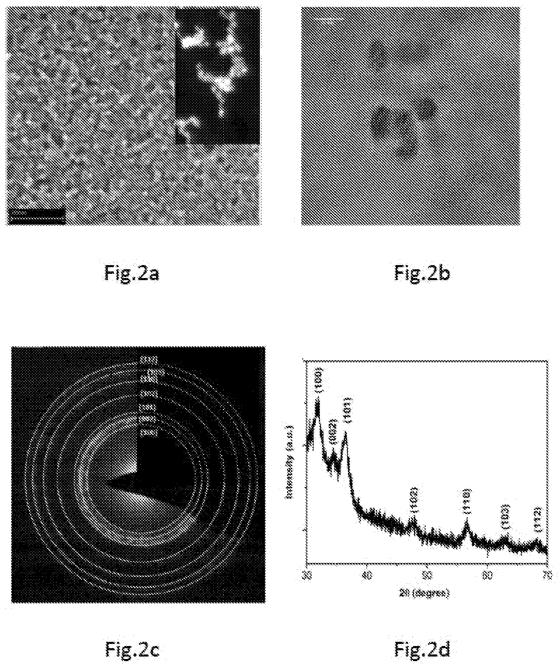

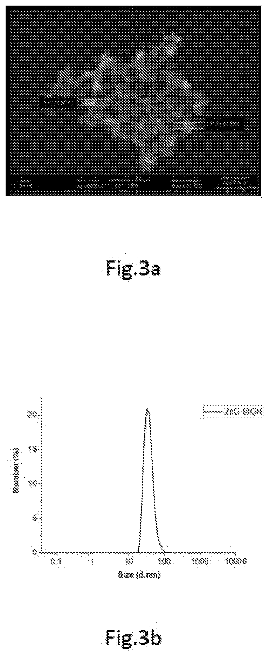

The zinc oxide nanocrystals were synthesized by means of the use of a microwave source. Thanks to this technology, it has been possible to obtain nanoparticles being spherical-shaped and monocrystalline in wurtzite phase with a homogeneous size distribution equal to approximately 20 nm, as shown in Figures 2a, 2b, 2c, 2d, 3a and 3b. The zinc oxide nanocrystals can remain dispersed in a colloidal manner (hydrodynamic diameter) in various solutions such as methanol (used in the synthesis), ethanol, water or polyethylene glycol, as shown in Figure 4.

### EXAMPLE 2

### Pre-treatment of zinc oxide to prepare it for subsequent doping.

In order to subsequently bind a dye to ZnO and, for example, amplify its fluorescence radiation, a functionalization of its surface has been carried out with amino groups (-NH₂), that are reactive against specific dye molecules, which otherwise would not bind exclusively to the oxide structure. The reaction takes place, in particular, between the ZnO nanocrystals and a reagent called aminopropyl-trimethoxy silane (APTMS) pouring all the components in a pyrex glass flask under nitrogen atmosphere with an anhydrous solvent (e.g. ethanol), stirring at least at 200 rpm with magnetic stir bar, and bringing the whole to boil for a period ranging between 5 and 8 hours, with the addition of a cooling column in order to avoid the solvent evaporation.

### EXAMPLE 3

### Extraction of exosomes from cancer cells.

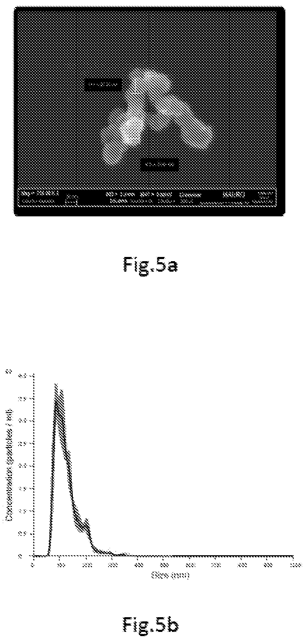

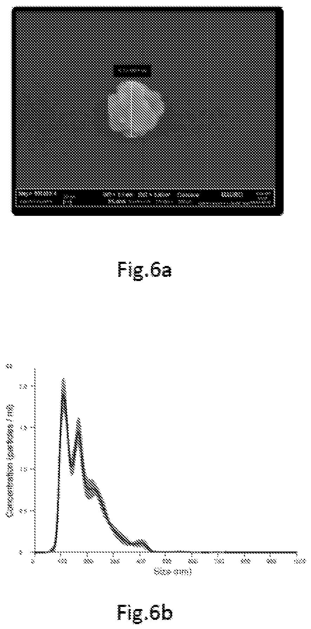

The exosome vesicles were produced from cancer cells of epithelial oral cancer (KB type, Figures 5a, 5b) and lymphoma cells (Daudi type, Figures 6a, 6b). The cells are cultured in a complete culture medium (Eagle's Minimum Essential Medium, EMEM or Roswell Park Memorial Institute, RPMI-1640, complete with 10% foetal bovine serum) for 48 h, after which the medium is replaced with one compound of EMEM or RPMI-1640 and 10% foetal bovine serum, from which the bovine exosomes have been previously removed (to prevent contamination). After 24-48 hours, the exosomes are extracted directly from such cell culture medium by means of differential ultracentrifugation techniques.

The exosomes obtained have a size ranging from 30 to 100 nm and were visualized either by scanning electron microscopy (Figures 5a, 6a) or by the Nanoparticle Tracking Analysis (NTA) technique (Figures 5b, 6b).

### EXAMPLE 4

### Coupling between exosomes and zinc oxide nanocrystals.

The coupling between exosomes and zinc oxide occurred by incubation of both in water and PBS (1:1 vol, Vₜₒₜₐₗ 100 µL) for certain time (1 h 30 min) at constant room temperature, and under stirring using a 250 rpm orbital shaker, followed by appropriate washings, centrifugation at 16870 g for 5 minutes, washing in a PBS:H₂O = 1:1 solution(Vₜₒₜₐₗ 100 µL), and purification processes to separate the exosomes and non-coupled ZnO from the final nanoassembly. The coupling process was then repeated on the supernatant portion (typically containing only exosomes) with a new aliquot of ZnO nanocrystals, followed by an appropriate washing.

### EXAMPLE 5

### Verification of coupling occurred between exosomes and zinc oxide nanocrystals.

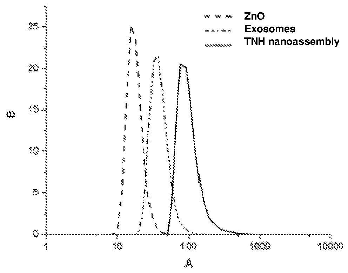

Verifications on the prepared materials (ZnO and exosomes), as well as on the coupling occurred into the nanoassembly, were conducted by means of various characterization techniques, including Dynamic Light Scattering (DLS, Figure 7). Thanks to such technique, it was possible to see how the diameter of the particles obtained has a larger size than individual exosomes and ZnO nanocrystals as such. In addition, by fluorescence microscopy (not shown in the Figure), after suitably marking nanocrystals with a dye emitting in the red wavelength, and exosomes with a dye emitting in the green wavelength, it was possible to acquire an image with the two different fluorescence channels (red and green) and superimpose the two images, evaluating the coupling rate between ZnO and the exosomes.

### EXAMPLE 6

### Coupling between monoclonal antibodies and nanoassembly lipid shell obtained in Example 4.

The coupling between the monoclonal antibody and the lipid shell was performed by means of antibody fusion and diffusion/fusion (suitably bound to a bifunctional polyethylene glycol spacer and a lipid molecule) into the lipid exosomial membrane.

### EXAMPLE 7

### Verification of radical species.

The measurement of oxidizing radical species generated by ZnO alone, or by the nanoassembly as a whole, was performed after the immersion of a material sample in water or buffered saline solutions or cell culture media, after internalisation for at least 24 hours in cancer cells. Successively, a paramagnetic resonance electronic spectrometer was used, capable of detecting the presence of free radicals generated by using a suitable chemical trap (5,5-dimethyl-1-pyrroline N-oxide, DMPO) capable of keeping them stable for a time sufficient for the measurement.

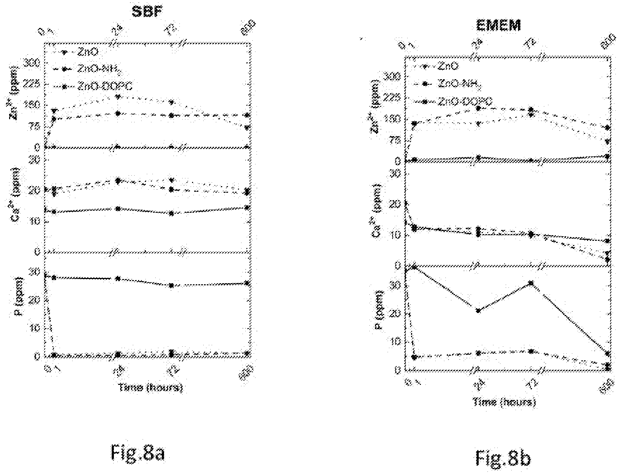

The measurement of zinc ions released from only from ZnO alone or from the whole nanoassembly was, therefore, carried out with ICP (Inductive Coupled Plasma)-Mass techniques (Figures 8a and 8b) and colorimetric methods in fluorescence with probes that bind specifically to zinc ions either in a cellular or non-cellular environment.

### EXAMPLE 8

### Testing of the imaging properties of the nanoassembly

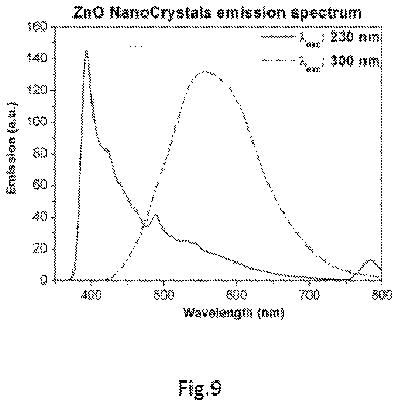

Imaging properties of ZnO and the nanoassembly as a whole have been investigated and measured by means of spectroscopy and fluorescence microscopy, presenting an emission centred at 500-600 nm in wavelength (Figure 9).

### EXAMPLE 9

### Cell viability tests after exposure to nanoassembly.

Cultured cells were exposed to the nanoassembly, and correct times of internalization were calibrated in cells (it resulted in the 62% of the cells of the KB line, which have internalized the nanoassembly after 5 hours, the 98% of cells which have internalized the nanoassembly after 24 hours), as well as the targeting specificity and the adequate doses of nanoassembly to be administered. Within the cell, but not externally, the nanoassembly loses its lipid coating and starts to develop radical species, and to dissolve into Zn^{2 +} ions, both leading to cell death.

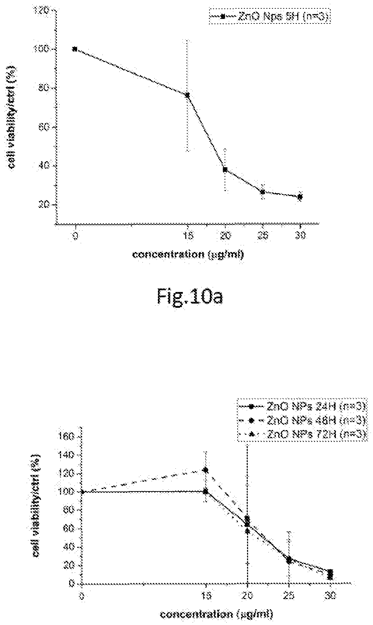

It has been found that, already at very low doses (15-20 µ g/ml) of zinc oxide nanocrystals in a cell culture medium, and already after just 5 hours after the administration, the cancer cells of the KB line die at a considerable rate (the survival rate of the cell population is lower than 40%), and such threshold decreases to 20% of survival rate for doses little higher than 25-30 µg/ml (Figures 10a and 10b), and is reduced to zero for dosages exceeding 30 µg/ml. The cell death was verified through various extensive testing of cell population counts, colorimetric (Trypan Blue and WST-1) as well as flow-cytometric methods, conventional in common biological practices.

### EXAMPLE 10

### Testing of the nanoassembly selectivity

The Anti-CD20 monoclonal antibody is used and coupled to the lipid shell of the nanoassembly. Such nanoassemblies are then cultured with leukemic cells (particularly, lymphomatous cancer cells of B, Daudi type) and, as a reference, with healthy B lymphocytes. It is demonstrated that the nanoassembly preferentiality and specific selectivity exist in binding by means of the Anti-CD20 antibody to the CD20-specific receptor on the Daudi cancer cell membrane. On the contrary, this selectivity does not exist when the nanoassembly with the AntiCD20 antibody is incubated with healthy B lymphocytes.

## Claims

1. A nanoassembly (1) for inducing apoptosis in cancer cells comprising:
- a core (2) comprising at least one nanoparticle of a nanostructured and semiconductor metal oxide, said nanoparticle being monocrystalline or polycrystalline;
- a shell (3) formed by a double phospholipid layer, proteins and other molecules, said shell (3) consisting of an extracellular biovesicle derived from cells of the same organism which said cancer cells belong to, said core (2) being enclosed inside said shell (3); and
- a plurality of targeting molecules (4, 4', 4") of said cancer cells, said molecules (4, 4', 4") being anchored to the external surface of said at least one biovesicle.

2. The nanoassembly (1) according to the preceding claim, wherein said targeting molecules (4, 4', 4") are monoclonal antibodies (4, 4', 4").

3. The nanoassembly (1) according to any of the preceding claims, wherein said nanoparticle is a sphere-shaped nanocrystal in wurtzite phase.

4. The nanoassembly (1) according to any of the preceding claims, wherein said metal oxide is zinc oxide.

5. The nanoassembly (1) according to any of the preceding claims, wherein said biovesicle is chosen among an exosome, an ectosome, a connectosome, an oncosome and an apoptotic body.

6. The nanoassembly (1) according to any claim 3 to 5, wherein the diameter of said at least one nanocrystal is comprised between 5 nm and 100 nm, and the diameter of said biovesicle is comprised between 30 nm and 300 nm.

7. The nanoassembly (1) according to any of the preceding claims, wherein the core (2) of said nanoassembly (1) comprises a molecule, including a medicament or a dye element having fluorescence properties.

8. The nanoassembly (1) according to any claim 4 to 7, wherein the zinc oxide is conjugated or doped with an element which broadens the fluorescence radiation in the ultraviolet-visible spectrum of said zinc oxide or modifies the frequency spectrum of said radiation.

9. The nanoassembly (1) according to the preceding claim, wherein said element, which modifies the spectrum, is adapted to broaden said spectrum up to comprise the frequencies in the infrared spectrum.

10. The nanoassembly (1) according to any claim 4 to 7, wherein the zinc oxide is doped with a paramagnetic or diamagnetic element, said element being adapted to be detected by means of a nuclear magnetic resonance scanner.

11. A kit for using the nanoassembly (1) as a contrast medium according to any claim 3 to 9, comprising:
- an injectable solution comprising said nanoassembly (1);
- means for emitting electromagnetic radiations in the ultraviolet spectrum by which radiating said nanoassembly (1);
- means for detecting the fluorescence radiation emitted by said nanoassembly (1) following the irradiation performed by said emitting means.

12. A method for manufacturing the nanoassembly (1) according to any claim 4 to 10, comprising the steps of:
- synthesis of the zinc oxide;
- *in vitro* culture of a plurality of cancer cells;
- extraction of a plurality of biovesicles from said plurality of cancer cells, said biovesicles having a diameter comprised between 30 nm and 300 nm;
- coupling of the biovesicles with the zinc oxide; and
- separation of the biovesicles coupled to the zinc oxide, remained non-coupled biovesicles and remained non-coupled zinc oxide.

13. The method according to the preceding claim, wherein the coupling of the biovesicles with the zinc oxide comprises the incubation of both in a solution obtained by mixing water and a phosphate-buffered saline with a volume ratio of 1:1.

14. The method according to the preceding claim, wherein the incubation is performed for a period comprised between 30 minutes and two hours and 30 minutes, at a temperature comprised between the room temperature and 37 °C, in static conditions or under mechanical stirring generated by an orbital stirrer with a number of rotations comprised between 50 and 350 rpm.

## Patentansprüche

1. Eine Nanoanordnung (1) zur Induzierung einer Apoptose in Krebszellen, umfassend:
- einen Kern (2), umfassend mindestens ein Nanopartikel eines nanostrukturierten und halbleitenden Metalloxids, wobei das Nanopartikel monokristallin oder polykristallin ist;
- eine Schale (3), die aus einer doppelten Phospholipidschicht, Proteinen und anderen Molekülen gebildet wird, wobei die besagte Schale (3) aus einem extrazellulären Biovesikel besteht, das aus Zellen desselben Organismus stammt, zu dem die besagten Krebszellen gehören, wobei der besagte Kern (2) in der besagten Schale (3) eingeschlossen ist; und
- eine Vielzahl von Targeting-Molekülen (4, 4', 4") der besagten Krebszellen, wobei die besagten Moleküle (4, 4', 4") an der äußeren Oberfläche des besagten mindestens einen Biovesikels verankert sind.

2. Die Nanoanordnung (1) gemäß dem vorhergehenden Anspruch, wobei die Targeting-Moleküle (4, 4', 4") monoklonale Antikörper (4, 4', 4") sind.

3. Die Nanoanordnung (1) gemäß einem jeden der vorhergehenden Ansprüche, wobei das besagte Nanopartikel ein kugelförmiges Nanokristall in Wurtzitphase ist.

4. Die Nanoanordnung (1) gemäß einem jeden der vorhergehenden Ansprüche, wobei das besagte Metalloxid Zinkoxid ist.

5. Die Nanoanordnung (1) gemäß einem jeden der vorhergehenden Ansprüche, wobei das besagte Biovesikel ausgewählt wird aus einem Exosom, einem Ektosom, einem Konnektosom, einem Onkosom und einem apoptotischen Körper.

6. Die Nanoanordnung (1) gemäß einem jeden Anspruch 3 bis 5, wobei der Durchmesser des besagten mindestens einen Nanokristalls zwischen 5 nm und 100 nm liegt und der Durchmesser des besagten Biovesikels zwischen 30 nm und 300 nm liegt.

7. Die Nanoanordnung (1) gemäß einem jeden der vorhergehenden Ansprüche, wobei der Kern (2) der besagten Nanoanordnung (1) ein Molekül, einschließlich eines Medikaments oder eines Farbstoffelements mit Fluoreszenzeigenschaften, umfasst.

8. Die Nanoanordnung (1) gemäß einem jeden Anspruch 4 bis 7, wobei das Zinkoxid mit einem Element konjugiert oder dotiert ist, das die Fluoreszenzstrahlung im ultraviolett-sichtbaren Spektrum des besagten Zinkoxids verbreitert oder das Frequenzspektrum der besagten Strahlung verändert.

9. Die Nanoanordnung (1) gemäß dem vorhergehenden Anspruch, wobei das besagte Element, das das Spektrum verändert, geeignet ist, das besagte Spektrum zu erweitern, um die Frequenzen im Infrarotspektrum zu umfassen.

10. Die Nanoanordnung (1) gemäß einem jeden Anspruch 4 bis 7, wobei das Zinkoxid mit einem paramagnetischen oder diamagnetischen Element dotiert ist, wobei das besagte Element geeignet ist, mithilfe eines Kernspinresonanzscanners nachgewiesen zu werden.

11. Ein Kit zur Verwendung der Nanoanordnung (1) als Kontrastmittel gemäß einem jeden Anspruch 3 bis 9, umfassend:
- eine einspritzbare Lösung, die die besagte Nanoanordnung (1) enthält
- Mittel zum Ausstrahlen elektromagnetischer Strahlung im ultravioletten Spektrum, durch die die besagte Nanoanordnung (1) bestrahlt wird;
- Mittel zum Nachweis der Fluoreszenzstrahlung, die von der besagten Nanoanordnung (1) ausgestrahlt wird, nach der Bestrahlung durch die besagten ausstrahlenden Mittel.

12. Verfahren zur Herstellung der Nanoanordnung (1) gemäß einem jeden Anspruch 4 bis 10, das die folgenden Schritte umfasst:
- Synthese des Zinkoxids;
- *In-vitro*-Kultur einer Vielzahl von Krebszellen; Extraktion einer Vielzahl von Biovesikeln aus der besagten Vielzahl von Krebszellen, wobei die besagten Biovesikel einen Durchmesser zwischen 30 nm und 300 nm aufweisen;
- Kopplung der Biovesikel mit dem Zinkoxid; und
- Trennung der an das Zinkoxid gekoppelten Biovesikel, der verbliebenen nicht gekoppelten Biovesikel und des verbliebenen nicht gekoppelten Zinkoxids.

13. Das Verfahren gemäß dem vorhergehenden Anspruch, wobei die Kopplung der Biovesikel mit dem Zinkoxid die Inkubation beider in einer Lösung umfasst, die durch Mischen von Wasser und einer phosphatgepufferten Salzlösung in einem Volumenverhältnis von 1:1 erhalten wird.

14. Das Verfahren gemäß dem vorhergehenden Anspruch, wobei die Inkubation über einen Zeitraum zwischen 30 Minuten und zwei Stunden und 30 Minuten bei einer Temperatur zwischen Raumtemperatur und 37°C unter statischen Bedingungen oder unter mechanischem Rühren mit einem Orbitalrührer mit einer Umdrehungszahl zwischen 50 und 350 U/min durchgeführt wird.

## Revendications

1. Nano-ensemble (1) pour induire l'apoptose dans les cellules cancéreuses comprenant :
- un noyau (2) comprenant au moins une nanoparticule d'un oxyde métallique nanostructuré et semi-conducteur, ladite nanoparticule étant monocristalline ou polycrystalline ;
- une enveloppe (3) comprenant une double couche de phospholipides, des protéines issues d'au moins une biovésicule extracellulaire, ledit noyau (2) étant enfermé à l'intérieur de ladite enveloppe (3) ; et
- une multitude de molécules de ciblage (4, 4', 4") desdites cellules cancéreuses, lesdites molécules (4, 4', 4") étant ancrées à la surface externe de ladite au moins une biovésicule.

2. Nano-ensemble (1) selon la revendication précédente, dans lequel lesdites molécules de ciblage (4, 4', 4") sont des anticorps monoclonaux (4, 4', 4").

3. Nano-ensemble (1) selon l'une des revendications précédentes, dans lequel ladite nanoparticule est un nanocristal en phase wurtzite.

4. Nano-ensemble (1) selon l'une des revendications précédentes, dans lequel l'oxyde métallique est l'oxyde de zinc.

5. Nano-ensemble (1) selon l'une des revendications précédentes, dans lequel ladite biovésicule est choisie parmi un exosome, un ectosome, un connectosome, un oncosome et un corps apoptotique.

6. Nano-ensemble (1) selon l'une des revendications 3 à 5, dans lequel le diamètre dudit au moins un nanocristal est compris entre 5 nm et 100 nm, et le diamètre de ladite biovésicule est compris entre 30 nm et 300 nm.

7. Nano-ensemble (1) selon l'une des revendications précédentes, dans lequel le noyau (2) dudit nano-ensemble (1) comprend une molécule, y compris un médicament ou un élément colorant ayant des propriétés de fluorescence.

8. Nano-ensemble (1) selon l'une des revendications 4 à 7, dans lequel l'oxyde de zinc est conjugué ou dopé avec un élément qui élargit le rayonnement de fluorescence dans le spectre ultraviolet-visible dudit oxyde de zinc ou modifie le spectre de fréquence dudit rayonnement.

9. Nano-ensemble (1) selon la revendication précédente, dans lequel ledit élément, qui modifie le spectre, est adapté pour élargir ledit spectre jusqu'à comprendre les fréquences dans le spectre infrarouge.

10. Nano-ensemble (1) selon l'une des revendications 4 à 7, dans lequel l'oxyde de zinc est dopé avec un élément paramagnétique ou diamagnétique, ledit élément étant adapté pour être détecté au moyen d'un scanner à résonance magnétique nucléaire.

11. Kit pour l'utilisation du nano-ensemble (1) comme agent de contraste selon l'une des revendications 3 à 9, comprenant :
- une solution injectable comprenant ledit nano-ensemble (1) ;
- des moyens d'émission de rayonnements électromagnétiques dans le spectre ultraviolet par lesquels rayonne ledit nano-ensemble (1) ;
- des moyens de détection du rayonnement de fluorescence émis par ledit nano-ensemble (1) suite à l'irradiation réalisée par lesdits moyens d'émission.

12. Procédé de fabrication du nano-ensemble (1) selon l'une des revendications 4 à 10, comprenant les étapes suivantes :
- synthèse de l'oxyde de zinc ;
- culture *in vitro* d'une multitude de cellules cancéreuses ;
- extraction d'une multitude de biovésicules à partir de ladite multitude de cellules cancéreuses, lesdites biovésicules ayant un diamètre compris entre 30 nm et 300 nm ;
- couplage des biovésicules avec l'oxyde de zinc ; et
- séparation des biovésicules couplées à l'oxyde de zinc, des biovésicules non couplées restantes et de l'oxyde de zinc non couplé restant.

13. Méthode selon la revendication précédente, dans laquelle le couplage des biovésicules avec l'oxyde de zinc comprend l'incubation des deux dans une solution obtenue en mélangeant de l'eau et une solution saline tamponnée au phosphate dans un rapport de volume de 1:1.

14. Méthode selon la revendication précédente, dans laquelle l'incubation est effectuée pendant une période comprise entre 30 minutes et deux heures et 30 minutes, à une température comprise entre la température ambiante et 37 °C, dans des conditions statiques ou sous agitation mécanique générée par un agitateur orbital avec un nombre de rotations compris entre 50 et 350 tr/min.
